# EUROPEAN PATENT SPECIFICATION

(11) **EP 0 935 955 B1**
(45) Date of publication and mention of the grant of the patent: **30.05.2007**
(21) Application number: 99103063.6
(22) Date of filing: 16.02.1999
(51) Int. Cl.: A61F 13/00

(54) **Application of adhesive to absorbent article**
Verfahren zum Anbringen von Klebstoffen auf einen absorbierenden Gegenstand
Procédé pour appliquer un matériau adhésif sur un article absorbant

(30) Priority: 17.02.1998 US 74890 P; 28.01.1999 US 239466
(43) Date of publication of application: 18.08.1999
(73) Proprietor: McNEIL-PPC, Inc., Skillman, New Jersey 08558 (US)
(72) Inventor: Luizzi, Joseph Michael Jr., Newton, PA 18940 (US)
(74) Representative: Metten, Karl-Heinz

(56) References cited:
- EP-A- 0 295 694
- WO-A-96/25902
- US-A- 4 718 898
- US-A- 4 854 995

## Description

### FIELD OF THE INVENTION

The present invention relates to a method of preparing a disposable absorbent article comprising a fluid impermeable adhesive coating; and articles made therefrom. In particular, the present invention discloses a method of applying an adhesive to a laminated topsheet and absorbent structure through the use of two non-contact coating dispensers in series, to be used as both the fluid impermeable containment and the positioning adhesive; thereby, eliminating the need for an additional containment layer.

### BACKGROUND OF THE INVENTION

Conventional disposable absorbent articles comprise a fluid permeable topsheet, an absorbent structure, a fluid impermeable backsheet as a containment layer, construction means for adhering the separate components, and an adhesive coating on the backsheet for attaching the disposable absorbent article to undergarments. Numerous variations of, and elements in addition to, these basic components have been described in an effort to either improve the disposable absorbent article's performance, or to reduce the cost of preparing the article. US Patent Number 4,718,898 discloses a napkin with a fluid impermeable backsheet comprised of a hot melt adhesive. The patent discloses that the hot melt adhesive can perform different functions in addition to serving as a containment layer. One such function is as positioning adhesive to attach the napkin to the crotch of undergarments. The adhesive disclosed in this patent is required to be at least about 1.0 mils thick in order to render the sanitary napkin fluid impermeable. The examples in the patent demonstrate that a thickness as low as 1.2 mils resulted in no observed leakage after twenty-four hours. Typical hot melt adhesives have density values in the range between about 0.98 to 1.06 grams per cubic centimeter. This would mean that the 1.2 mils coat thickness in US 4,718,898 would result in a coating basis weight range of about 25 to 30 gsm. PCT application WO 96/25902 describes a coating method that employs a non-contact application wherein a thermoplastic composition is thermally made flowable and released from a coating device onto a substrate. The thermoplastic composition is thus coated onto the substrate without contact between said coating device and said substrate. The method employs a single dispenser to coat the product. Thus in the manufacture of the product there is only one opportunity to produce an impermeable containment barrier layer free of voids or occlusions. Also, WO 96/25902 demonstrates significant rheological and complex viscosity limitations. The method as described would not work effectively with adhesives that are typically used as a construction adhesives, namely those based on styrene copolymers. The coating process would either require higher coat weights, or slower speeds, in order to produce a fluid impermeable containment. In addition, typical adhesives that are used for attaching the pad for use fall outside the preferred rheological window and can not be successfully coated in this manner. Added coat weights and slower speeds are not commercially acceptable alternatives to current disposable manufacturing methods. Despite the disclosures cited herein, there remains a need for a simple non-contact coating method capable of producing a low basis weight, continuous coating layer, using a broad range of industry standard hot melt adhesives, to achieve a fluid impermeable containment adhesive layer for a disposable absorbent article.

### SUMMARY OF THE INVENTION

The present invention discloses a method for producing a disposable absorbent article comprising: providing a fluid impermeable topsheet, providing an absorbent structure, joining said coversheet and said absorbent structure to form a laminate with a body facing side and a garment facing side, providing a first means for coating the laminate, such that the first means for applying a first adhesive does not contact the laminate,
applying the first thermoplastic adhesive to said garment facing side, providing a second means for coating the laminate, such that the second means for applying a second adhesive does not contact the laminate, applying the second adhesive to said garment facing side of the laminate, thereby forming a disposable absorbent article in the absence of a containment layer, wherein at least one of the adhesives applied to the disposable absorbent article has a complex viscosity value greater than about 1,000 poise at a shear rate of about 10 rad. / sec. (radians per second), and the garment facing side of the laminate has a combined coating basis weight of less than about 20 gsm.; rendering the disposable absorbent article substantially fluid impermeable. A second embodiment of the present invention is a disposable absorbent article comprising a fluid impermeable adhesive coating produced from the method disclosed above.

### DETAILED DESCRIPTION OF THE PREFERRED EMBODIMENTS

The present invention discloses a method of applying a first and a second adhesive coating to a laminated top sheet and absorbent structure, to be used as both the fluid impermeable containment layer and the positioning adhesive; thereby, eliminating the requirement of a fluid containment barrier, such as a thermoplastic film. Conventional processes used to produce disposable absorbent articles contain two adhesive dispensers on-line: one for applying construction adhesive needed to adhere the absorbent structure and topsheet laminate to a thermoplastic containment layer, and the second for applying a positioning adhesive to the outside surface of the containment layer. A typical type of adhesive dispenser used on such equipment is a slot nozzle. One of the existing adhesive dispensers is eliminated by virtue of the present invention, thus allowing for the series of dispensers needed for a multiple containment / positioning adhesive coating application as described in the present invention.

The present invention is a method comprising coating a laminate of an absorbent structure and a fluid permeable topsheet with adhesives having complex viscosity values greater than about 1,000 poise at about 10 rad. / sec., and then coating the laminate a second time with similar adhesives for a combined coating weight of less than or equal to about 20 grams per square meter (gsm), thereby making an disposable absorbent article that is substantially fluid impermeable. Since this method utilizes two or more coating dispensers, neither the first coating nor subsequent coatings need to be fluid impermeable in, and of themselves. It is the composite coating produced from two or more adhesive layers that needs to be fluid impermeable. If any voids are produced and exist in an individual coating layer, coatings that precede and/or are applied subsequently will occlude the void, thus rendering the entire composite coating of layers impermeable to fluid.

Most positioning and construction adhesive compositions known in the art can be employed in the present invention. Suitable adhesives include, but are not limited to, acrylics, starch based hot melts, adhesives based on block copolymers of vinyl aromatic hydrocarbon and one or more conjugated diene or hydrogenated aliphatic blocks, polylactic acids, hot melts based polyolefins such as amorphous poly alpha olefins which may consist of one or more of the following monomers: propylene, ethylene, butene, and hexene, hot melts based on low density polyethylene or low density polyethylene copolymers including ethylene vinyl acetate, methyl acrylate, n-butyl acrylate, and acrylic acid.

Also, adhesives with certain properties are suitable for incorporation into the present invention. Such adhesives include, but are not limited to, those that are water soluble, water dispersible, biodegradable, hydrophilic, hydrophobic, and highly resilient to deformation, also known in the art as bunching or twisting, which relates to the article losing its desired shape.

Suitable biodegradable adhesives include, but are not limited to, the following: starch based, hydroxy alkyl cellulose based, polylactic acid based, polyethylene oxazoline copolymer, and polyethylene oxide vinyl acetate grafted copolymer. In one embodiment of the present invention, the first and second adhesive coatings are the same. This embodiment allows for the advantages of compatibility of the two coatings. In a second embodiment, the first and second adhesive coatings are different. In this embodiment, the first adhesive coating can be selected to have good compatibility to the substrate being coated, especially when the substrate is fibrous in nature such as a nonwoven laminate. The nonwoven laminate can be made up of different types of fibers and other materials such as cellulosics, bicomponent fibers, polyesters, wood pulp, absorbent polymers, and the like.

A further advantage of using two different coatings is that the first coating can aid in the-management of fluid, i.e. the first adhesive is hydrophilic in nature, while the second coating is used primarily as a positioning adhesive. Hydrophilic hot melt adhesives are commercially available and noted in the literature to provide enhancements in fluid management. Examples of this type of adhesive include Hydromelt HL-1614 from H.B Fuller, Aquireg H-4117 from ATO-Findley and Cycloflex^{™} 70-4651 from National Starch & Chemical. These adhesives are hydrophilic in nature but are not water soluble. Thus, they maintain their integrity in use. A disposable absorbent article can thus be constructed using a fluid impermeable adhesive composite in which the first adhesive coated onto the absorbent laminate is hydrophilic, and the last layer is a typical positioning adhesive. Typical positioning adhesives that are well known in the art are based styrenic block copolymers as disclosed in US patent numbers 5,149,741; 5,143,968; and 5,057,571. The hydrophilic inner layer will aid in fluid transport moving fluid along the impermeable containment through the absorbent media.

Still a further advantage of using two different adhesive coatings, is that the first coating can be highly resilient, while the second adhesive coating is primarily used as the positioning adhesive. Examples of adhesives that have high resilient properties are, but not limited to the following: adhesives that are high in elastomer content like Fullastics adhesives from H.B. Fuller, or high modulus adhesives like ASI-1261 from Adhesives Systems Incorporated, or HL-7500 from H.B. Fuller, or 34-2952 from National Starch and Chemical. The finished article has improved resiliency and is therefore less prone to bunching; a major consumer complaint category for external sanitary protection products. Suprisingly, adhesives with complex viscosities higher than about 1,000 poise, at a shear rate of about 10 rad. / sec., can be incorporated into this invention, while maintaining high processing speeds. Preferably from about 1, 100 to 10,000 poise, at a shear rate of 10 rad. sec. And most preferably, from about 1,200 to about 7,500 poise, at a shear rate of 10 rad. / sec. As used throughout application, complex viscosity is defined as measured by dynamic mechanical analysis, using such instruments as a Rheometics RSA II, at temperatures in between about 90 and 120 degrees Celsius. Each adhesive sample was tested as a function of frequency from about 0. 1 to 100 rad. / sec. at various isothermal conditions from about 60 to 150 degrees Celsius. Data was then shifted in accordance to the Williams-Landol-Ferry polymer superposition principle about a single reference temperature. In this way, data can be obtained outside the limitations to the current instrumentation in the 10³ to 10⁶ rad. / sec. range. In addition to the ability to use high complex viscosity adhesives, the present invention allows for higher processing speeds. Conventional equipment used to prepare disposable absorbent articles process at speeds in excess of about 600 feet a minute. Preferably processing speeds are from about 3,18 m/s to about 5,08 m/s [625 to about 1,000 feet a minute]. Most preferably the processing speeds are from about 3,30 m/s to about 3,81 m/s [650 to 750 feet a minute].

The total coating thickness should be no more than about 30 gsm. Typically from about 10 to 25 gsm. And preferably from about 15 to 20 gsm. Thicker coatings can be employed to render the article fluid impermeable, but at the expense of the adhesives being applied. The ratio of the two adhesive coatings is not critical. Preferably the first and second adhesive coatings are applied in substantially equal amounts; however, in order to improve certain properties of a disposable absorbent article, it may be desirable to incorporate a thicker layer of first adhesive coating and a thinner layer of second adhesive coating.

Non -contact coating techniques that can suitably be used in the present invention include, but are not limited to slot, dip, spray, melt blown, control coat available from Nordson, and swirl spray. Preferably a slot nozzle is employed to apply the adhesives in the present invention.

The present invention is directed to two or more adhesive coating applications performed in series, with at least a portion of the second adhesive coating contacting the first adhesive coating. Preferably more than about 40 % of the first coating is in contact with the second coating. Most preferably more than about 70 % of the first coating is in contact with the second coating.

Another embodiment is the coating means apply the adhesives to different locations, so long as the article remains substantially fluid impermeable. For example, the first coating can cover the central portion of an absorbent structure without extending completely to the longitudinal edges and subsequent coatings can cover the entire surface area of the structure. This would render the central portion of the absorbent structure substantially impermeable to fluid, while the longitudinal edges of the structure have an adhesive containment layer that may or may not be impermeable to fluid due to the coatings not overlapping in this region. Other patterns that include non-overlapped regions such as stripes, islands, ovals, and the like can be employed to assist in customization of the article. In addition, the adhesive coatings can be applied discontinuously, so long as the article remains substantially fluid impermeable. The non-overlapped regions could then allow the absorbent article to breathe. Breathability is described by a value of air permeability measured on a Frazier Air Permeometer. One method for measuring air permeability ("breathability") of nonocclusive structures is the Frazier Air Permeometer. Air permeability is the volume rate of airflow passing through a square foot sample of structure, in cubic feet per minute, that is necessary to maintain an air pressure across the sample of 0.5 inch of water. The Frazier Air Permeometer (Frazier Precision Instrument Company, Inc., Hagerstown, MD) indirectly determines this airflow by measuring the pressure drop across the sample in the test chamber through which air is passing. A calibration curve is used to convert the pressure drop readings to air permeability. A sample is clamped in place a the entrance to a first chamber which communicates through an orifice to a second chamber wherein the air flow differential is created by means of a suction fan, the size of the orifice being appropriate to the anticipated air permeability range to be measured. The suction is increased to the point where the pressure in the first chamber as recorded on an inclined oil manometer is about 0.5 inch of water. The pressure in the second chamber as recorded on a vertical oil manometer and is converted to air permeability by means of a conversion chart.

The order of application of the two dimensionally different adhesive coatings would not affect the result of the described embodiment. Similarly, a disposable absorbent article could be prepared in a transverse direction, with respect to the machine direction, resulting in the non-overlapped regions of adhesive to fall on the lateral edges instead of the longitudinal edges. In addition to the breathability benefits potentially gained from this embodiment adhesives with varying degrees of resiliency can be applied to different regions of the absorbent article to provide stability during use.

Convenience and environmental concern are both important benefits to consumers who use disposable absorbent articles. One of the cited conveniences that consumers would like is to be able to dispose of a soiled absorbent article in a toilet and flush it away without clogging the toilet. An approach that improves the ability of an article to successfully clear a toilet bowl and waste sewage pipes, is to decrease the size of the article. The size of the absorbent article can be decreased by dispersion it into many smaller pieces. Using water dispersible / soluble adhesives makes it possible for the article to disperse when immersed in a toilet's water. The present invention can further improve the flushability of a disposable absorbent article through the elimination of the containment layer. Examples of water dispersible / soluble adhesives are the following: Cycloflex^{™} 70-4073 adhesives from National Starch and Hydromelt HL-1600 from H.B. Fuller. Whether or not a disposable absorbent article is disposed of in a waste container or in a toilet, consumers are concerned with the impact on the environment. There are commercially available thermoplastic adhesives that are biodegradable. The present invention is clearly an advantage over the prior art, which discloses serious rheological and complex viscosity limitations for the thermoplastic structures being used. Certain types of adhesives typically used in the industry for pad attachment do not fall within the narrowly outlined rheological and complex viscosity windows disclosed in the art; or process tolerances necessary to fit the structure within the claimed theological window, such as temperature are too narrow to be commercially viable. By contrast, the current method offers a more robust manufacturing process.

Those that are skilled in the art will appreciate that more than two adhesive coatings can be applied to an absorbent article. This would allow a customization of the adhesive coatings for various tasks. For example, the first coating could be hydrophilic to assist in fluid management. The second coating could be resilient to bunching. A third coating could be used as positioning adhesive. Other combinations are readily apparent to those skilled in the art. In addition, those skilled in the art will recognize that if a positioning adhesive is employed, it will typically be the last coating applied to the article.

Another embodiment of the present invention is a disposable absorbent article with a fluid impermeable adhesive coating, that is less than about 30 gsm in weight, to be used as both a containment layer and the positioning adhesive. Examples of disposable articles include pantyliners, napkins, incontinent products, diapers, bed pads, surgical drapes, and wound dressings. Conventional disposable absorbent articles are generally composed of a fluid permeable topsheet, which is designed to draw fluid away from the site of contact. An absorbent structure is found in the next layer, and this absorbent structure is designed to absorb fluids as they pass through the topsheet. Such absorbent articles often have a substantial absorption capacity. A fluid impermeable backsheet is then attached to the absorbent structure using some type of hot melt adhesive. This backsheet acts as a containment to prevent fluid from leaking out of the absorbent media. In the case of sanitary pads, panty liners, and adult incontinence products, a pressure sensitive adhesive may then be coated onto the containment, so that the product may be attached to the crotch of the undergarment for use. The process traditionally employed in the manufacture of sanitary products involves a series of manufacturing steps where the topsheet, containment, and sometimes the absorbent structure are taken in as rolled goods, which are then laminated together using hot melt adhesives, and coated with a positioning adhesive and protective release coating, which may be apart of the packaging.

The disposable absorbent articles described above can me manufactured into various geometric shapes. The article can be substantially rectangular in shape, or can have a contour shape similar to an hourglass. The article has lateral edges and longitudinal edges, with the longitudinal direction of the article being greatest in dimension. The longitudinal edges can be extended or have extensions attached that provide additional absorption. Typically the extensions are folded around the undergarment and attached to the outside surface of the undergarment.

As used herein, fluid impermeability is understood to be measured using the following method. A stack of four 4 x 8 inch sheets of Whatman Number I Qualitative filter paper is weighed. The test sample comprising a test disposable absorbent article is placed with its adhesive layer downward in contact with the top sheet of the filter paper stack. A Plexiglass acrylic plate, 5,08 cm [2 inches] wide, 20,32 cm [8 inches] long, and 1,11 cm [7/16 inches] thick and containing an orifice 1,27 cm [0.5 inch], for admitting fluid is placed on top of the disposable absorbent article. Two 2 kilogram weights are placed on either side of the orifice and 1.3 milliliters red dyed 0.9% saline solution is poured through the orifice at an even rate. One minute after the fluid has been completely added, the filter paper is weighed to determine how much fluid, if any, has leaked through the adhesive layer. Substantially fluid impermeable would then be defined as the filter paper increasing in weight by less than 0. 13 grams.

## Claims

1. A method for producing a disposable absorbent article comprising
a) providing a fluid-permeable topsheet,
b) providing an absorbent structure,
c) joining said topsheet and said absorbent structure to form a laminate with a body facing side and a garment facing side,
d) providing a first means for coating the laminate, such that the first means for applying a first thermoplastic adhesive does not contact the laminate,
e) applying the first thermoplastic adhesive to said garment facing side of the laminate,
f) providing a second means for coating the laminate, such that the second means for applying a second thermoplastic adhesive does not contact the laminate,
g) applying the second thermoplastic adhesive to said garment facing side of the laminate, thereby forming a disposable absorbent article in the absence of a thermoplastic containment layer, wherein at least one of adhesives applied to the article has a complex viscosity value greater than about 1,000 poise at a shear rate of about 10 radians/second, and the garment facing side of the laminate has a combined adhesive coat weight of less than about 20 grams square meter, rendering the disposable absorbent article substantially fluid impermeable.

2. The method of claim 1, wherein both the first and second adhesives are **characterized** with complex viscosity values greater than about 1,000 poise at a shear rate of about 10 radians /sec.

3. The method of claim 1, wherein the laminate speed is greater then about 3,05 m/s [600 feet a minute].

4. The method of claim 1, wherein the first and second adhesives are different in composition.

5. The method of claim 4 wherein in least one of the applied adhesive coatings is **characterized** with complex viscosity values greater than about 1,000 poise at a shear rate about of 10 radians / second.

6. A method for producing a disposable absorbent article comprising
a) providing an absorbent structure with a first and second side,
b) providing a first means for coating the absorbent structure, such that the first means for applying a first adhesive does not contact the structure,
c) applying the first adhesive to said second side of the absorbent structure,
d) providing a second means for coating the absorbent structure, such that the second means for applying a second adhesive does not contact the structure,
e) applying the second adhesive to said second side of the absorbent structure, thereby forming an absorbent article in the absence of a thermoplastic containment layer, wherein at least one of the adhesives applied to the disposable absorbent structure has a complex viscosity value grater than about 1,000 poise at a shear rate of about 10 radians / second, and the absorbent structure having a combined adhesive coat weight of less than 20 gram square meter, rendering the disposable absorbent article substantially fluid impermeable.

7. The method of claim 6 wherein the adhesives are not of the same composition.

8. A disposable absorbent article comprising a fluid impermeable adhesive coating produced using a method according to claim 1.

9. The article of claim 8 wherein the adhesive coatings are substantially contiguous with one another.

10. A disposable absorbent article comprising a fluid impermeable adhesive coating produced using a method according to claim 6.

## Patentansprüche

1. Verfahren zur Herstellung eines absorbierenden Wegwerfartikels, umfassend:
a) Bereitstellen einer flüssigkeitsdurchlässigen Decklage,
b) Bereitstellen einer absorbierenden Struktur,
c) Verbinden der Decklage und der absorbierenden Struktur, um ein Laminat mit einer dem Körper zugewandten Seite und einer der Kleidung zugewandten Seite auszubilden,
d) Bereitstellen eines ersten Mittels zum Beschichten des Laminats, so dass das erste Mittel zum Aufbringen eines ersten thermoplastischen Klebstoffs nicht in Kontakt mit dem Laminat gelangt,
e) Aufbringen des ersten thermoplastischen Klebstoffs auf die der Kleidung zugewandte Seite des Laminats,
f) Bereitstellen eines zweiten Mittels zum Beschichten des Laminats, so dass das zweite Mittel zum Aufbringen eines zweiten thermoplastischen Klebstoffs nicht in Kontakt mit dem Laminat gelangt,
g) Aufbringen des zweiten thermoplastischen Klebstoffs auf die der Kleidung zugewandte Seite des Laminats, wodurch ein absorbierender Wegwerfartikel in Abwesenheit einer thermoplastischen Rückhalteschicht ausgebildet wird, wobei zumindest einer der auf den Artikel aufgebrachten Klebstoffe einen komplexen Viskositätswert von über ca. 1.000 Poise bei einer Schergeschwindigkeit von ca. 10 Radiant/Sekunde hat und die der Kleidung zugewandte Seite des Laminats ein kombiniertes Klebstoffschichtgewicht von weniger als ca. 20 Gramm pro Quadratmeter hat, wodurch der absorbierende Wegwerfartikel im wesentlichen flüssigkeitsundurchlässig gemacht wird.

2. Verfahren nach Anspruch 1, wobei sowohl der erste als auch der zweite Klebstoff durch komplexe Viskositätswerte von über ca. 1.000 Poise bei einer Schergeschwindigkeit von ca. 10 Radiant/Sek. **gekennzeichnet** sind.

3. Verfahren nach Anspruch 1, wobei die Laminatgeschwindigkeit größer ist als 3,05 m/s [600 Fuß pro Minute].

4. Verfahren nach Anspruch 1, wobei sich der erste und zweite Klebstoff in ihrer Zusammensetzung unterscheiden.

5. Verfahren nach Anspruch 4, wobei zumindest eine der aufgebrachten Klebstofflaminate durch komplexe Viskositätswerte von über ca. 1.000 Poise bei einer Schergeschwindigkeit von ca. 10 Radiant/Sekunde **gekennzeichnet** ist.

6. Verfahren zur Herstellung eines absorbierenden Wegwerfartikels, umfassend:
a) Bereitstellen einer absorbierenden Struktur mit einer ersten und zweiten Seite,
b) Bereitstellen eines ersten Mittels zum Beschichten der absorbierenden Struktur, so dass das erste Mittel zum Aufbringen eines ersten Klebstoffs nicht in Kontakt mit der Struktur gelangt,
c) Aufbringen des ersten Klebstoffs auf die zweite Seite der absorbierenden Struktur,
d) Bereitstellen eines zweiten Mittels zum Beschichten der absorbierenden Struktur, so dass das zweite Mittel zum Aufbringen eines zweiten Klebstoffs nicht in Kontakt mit der Struktur gelangt,
e) Aufbringen des zweiten Klebstoffs auf die zweite Seite der absorbierenden Struktur, wodurch ein absorbierender Artikel in Abwesenheit einer thermoplastischen Rückhalteschicht ausgebildet wird, wobei zumindest einer der auf die absorbierende Wegwerfstruktur aufgebrachten Klebstoffe einen komplexen Viskositätswert von über ca. 1.000 Poise bei einer Schergeschwindigkeit von ca. 10 Radiant/Sekunde hat und die absorbierende Struktur ein kombiniertes Klebstoffschichtgewicht von weniger als ca. 20 Gramm pro Quadratmeter hat, wodurch der absorbierende Wegwerfartikel im Wesentlichen flüssigkeitsundurchlässig gemacht wird.

7. Verfahren nach Anspruch 6, wobei die Klebstoffe nicht dieselbe Zusammensetzung aufweisen.

8. Absorbierender Wegwerfartikel, der eine flüssigkeitsundurchlässige Klebstoffbeschichtung, hergestellt unter Verwendung eines Verfahrens nach Anspruch 1, umfasst.

9. Artikel nach Anspruch 8, wobei die Klebstoffbeschichtungen im wesentlichen einander benachbart sind.

10. Absorbierender Wegwerfartikel, der eine flüssigkeitsundurchlässige Klebstoffbeschichtung, hergestellt unter Einsatz eines Verfahrens nach Anspruch 6, umfasst.

## Revendications

1. Procédé de production d'un article absorbant jetable comprenant :
a) la mise à disposition d'une couche supérieure perméable aux fluides,
b) la mise à disposition d'une structure absorbante,
c) la réunion de ladite couche supérieure et de ladite structure absorbante pour former un stratifié avec une face dirigée vers le corps et une face dirigée vers les vêtements,
d) l'application d'un premier moyen pour revêtir le stratifié, tel que le premier moyen d'application d'un premier adhésif thermoplastique ne soit pas en contact avec le stratifié,
e) l'application du premier adhésif thermoplastique à ladite face dirigée vers les vêtements du stratifié,
f) l'application d'un second moyen pour revêtir le stratifié, tel que le second moyen d'application d'un second adhésif thermoplastique ne soit pas en contact avec le stratifié,
g) l'application du second adhésif thermoplastique à ladite face dirigée vers les vêtements du stratifié, pour former ainsi un article absorbant jetable en l'absence d'une couche de confinement thermoplastique, dans lequel au moins un des adhésifs appliqués à l'article a une valeur de viscosité complexe supérieure à environ 1 000 poise à une vitesse de cisaillement d'environ 10 radian/seconde, et la face dirigée vers les vêtements du stratifié a un grammage adhésif combiné de moins d'environ 20 grammes par mètre carré, rendant le dispositif absorbant jetable essentiellement imperméable aux fluides.

2. Procédé selon la revendication 1, dans lequel à la fois les premier et second adhésifs sont **caractérisés par** des valeurs de viscosité complexe supérieures à environ 1 000 poise à une vitesse de cisaillement d'environ 10 radian/seconde.

3. Procédé selon la revendication 1, dans lequel la vitesse du stratifié est supérieure à environ 3,05 m/s [600 pied par minute] .

4. Procédé selon la revendication 1, dans lequel les premier et second adhésifs sont de compositions différentes.

5. Procédé selon la revendication 4, dans lequel au moins un des revêtements adhésifs appliqués est **caractérisé par** des valeurs de viscosité complexe supérieures à environ 1 000 poise à une vitesse de cisaillement d'environ 10 radian/seconde.

6. Procédé de production d'un article absorbant jetable comprenant
a) la mise à disposition d'une structure absorbante avec une première et une seconde face,
b) l'application d'un premier moyen pour revêtir la structure absorbante, de sorte que le premier moyen d'application d'un premier adhésif ne soit pas en contact avec la structure,
c) l'application du premier adhésif à ladite seconde face de la structure absorbante,
d) l'application d'un second moyen pour revêtir la structure absorbante, de sorte que le second moyen d'application d'un second adhésif ne soit pas en contact avec la structure,
e) l'application du second adhésif thermoplastique à ladite seconde face de la structure absorbante, pour former ainsi un article absorbant en l'absence d'une couche de confinement thermoplastique, dans lequel au moins un des adhésifs appliqués à la structure absorbante jetable a une valeur de viscosité complexe supérieure à environ 1 000 poise à une vitesse de cisaillement d'environ 10 radian/seconde, et la structure absorbante ayant un grammage adhésif combiné de moins de 20 grammes par mètre carré, rendant le dispositif absorbant jetable essentiellement imperméable aux fluides.

7. Procédé selon la revendication 6 dans lequel les adhésifs ne sont pas de composition identique.

8. Article absorbant jetable comprenant un revêtement adhésif imperméable aux fluides produit en utilisant un procédé selon la revendication 1.

9. Article selon la revendication 8 dans lequel les revêtements adhésifs sont essentiellement contigus entre eux.

10. Article absorbant jetable comprenant un revêtement adhésif imperméable aux fluides produit en utilisant un procédé selon la revendication 6.
